# EUROPÄISCHE PATENTANMELDUNG

(11) **EP 2 033 683 A1**
(43) Veröffentlichungstag der Anmeldung: **11.03.2009**
(21) Anmeldenummer: 08161237.6
(22) Anmeldetag: 28.07.2008
(51) Int. Cl.: A61N 1/05

(54) **Elektrodenvorrichtung zum physiologischen Einsatz insbesondere in der Kardiologie**

(30) Priorität: 10.09.2007 DE 102007043127
(71) Anmelder: BIOTRONIK CRM Patent AG, 6341 Baar (CH)
(72) Erfinder: Kolberg, Gernot, 12043 Berlin (DE); Flach, Erhard, 12305 Berlin (DE); Schurr, Marc, 13359 Berlin (DE); Zedler, Stephan, 12309 Berlin (DE)
(74) Vertreter: Lindner-Vogt, Karin L.

(57) **Zusammenfassung**

Eine Elektrodenvorrichtung zum physiologischen Einsatz insbesondere in der Kardiologie weist einen elektrisch isolierenden, elastischen Elektrodenkörper auf, der zwischen einer Innen- und Außenschicht (1, 2) eine auf deren physikalische Eigenschaften abgestimmte Zwischenschicht (3) aufweist.

## Beschreibung

Die Erfindung betrifft eine Elektrodenvorrichtung zum physiologischen Einsatz insbesondere in der Kardiologie mit einem aus elektrisch isolierenden, elastischen Materialien schichtweise aufgebauten Elektrodenkörper.

Zum Hintergrund der Erfindung ist festzuhalten, dass das für einen praxisgerechten Elektrodenkörper, der beispielsweise in Herzgefäßen als Signalgeber von implantierten Herzschrittmachern eingesetzt wird, verschiedene Elektrodeneigenschaften, wie elektrische Isolation, mechanische Eigenschaften wie Biege- und Torsionsteifigkeit, Gleiteigenschaften und ein ausreichender Abriebschutz von Relevanz sind. Diese unterschiedlichen Eigenschaften stellen an die für den Elektrodenkörper verwendeten Werkstoffe unterschiedliche und teilweise widersprechende Anforderungen, wie an dem folgenden Praxisbeispiel deutlich wird. So werden Elektrodenkörper üblicher Weise aus physiologischen Gründen aus dem chemisch und elektrisch sehr dauerstabilen Silikon hergestellt. Dieses Material hat jedoch den Nachteil, dass es schlechte Gleiteigenschaften aufweist und ein hohes Abriebrisiko zeigt.

Aus dem Stand der Technik ist es in diesem Zusammenhang bekannt, zur Verbesserung der Gleiteigenschaften eine dünne Gleitschicht auf das Silikon aufzutragen. Andererseits kann zur Verbesserung der Abriebeigenschaften - und in gewissem Maße auch der Gleiteigenschaften - eine Polyurethanschicht auf dem Silikonkörper vorgesehen werden. Unter "Schicht" ist dabei - was für die weitere Offenbarung der Erfindung durchgehend zutreffen soll - ein separater Schlauch, eine Hülle oder eine auf ein tragendes Schichtteil aufgebrachte Beschichtung zu verstehen.

Der Nachteil der vorstehenden Lösungsansätze für die Verbesserung der Gleit- und Abriebeigenschaften liegt darin, dass die Kombination der angegebenen Werkstoffe wegen ihrer unterschiedlicher Eigenschaften an der Grenzfläche zum jeweils anderen Material mechanische Spannungen hervorruft, die ein Ausmaß annehmen können, so dass im Dauereinsatz der Elektrodenvorrichtung mindestens einer der beiden Materialpartner geschädigt wird. So kann beispielsweise eine sehr dünne, durch Fluorierung erzeugte, harte Gleitbeschichtung eines Silikon-Isolationsschlauches des Elektrodenkörpers sehr feine Oberflächenrisse bekommen, die sich in das Silikonmaterial fortpflanzen und damit die Zugfestigkeit des Silikons herabsetzen können.

Bei einer Materialpaarung von Polyurethan auf Silikon kann auf Grund der Spannungen zwischen den Materialien die Haftung zwischen diesen beiden Materialpartnern beim Dauereinsatz verloren gehen. Dies führt bei einer Dauerbewegung der Elektrodenvorrichtung, wie sie beispielsweise bei einer in einem Herzgefäß implantierten, im Blutstrom floatenden Elektrode der Fall sein kann, zu einer Reibung zwischen den Materialpartnern und damit zu einem Abrieb des Silikons. Grundsätzliche Ursache für diese Nachteile, die insbesondere im Dauereinsatz einer Elektrodenvorrichtung zu einem Problem werden können, sind die zwischen den verschiedenen Werkstoffen auftretenden Spannungen.

Ausgehend von der geschilderten Problematik des Standes der Technik liegt der Erfindung die Aufgabe zu Grunde, einen aus elektrisch isolierenden, elastischen Materialien schichtweise aufgebauten Elektrodenkörper so auszubilden, dass die damit ausgerüsteten Elektrodenvorrichtungen im physiologischen Einsatz besonders dauerstabil sind.

Diese Aufgabe wird durch die im Kennzeichnungsteil des Anspruchs 1 angegebenen Merkmale gelöst, wonach
- zwischen einer Innenschicht und einer Außenschicht des Elektrodenkörpers ein Gradient der Werte einer physikalischen Eigenschaft der Schichten vorliegt, und
- zwischen Innenschicht und Außenschicht mindestens eine Zwischenschicht eingesetzt ist, die mindestens einen zwischen den Werten der Außen- und Innenschicht liegenden Zwischenwert dieser physikalischen Eigenschaft aufweist.

Der Erfindung liegt also der konzeptionelle Gedanke zu Grunde, die durch die unterschiedlichen Werte einer physikalischen Eigenschaft der Innen- und Außenschicht, wie also beispielsweise eine unterschiedliche Härte oder Elastizität, hervorgerufenen Spannungen in den Grenzflächen zu reduzieren, indem die Materialien unterschiedlicher Eigenschaften durch Materialien getrennt werden, deren Eigenschaften dazwischen liegen. Zwischen der Innen- und Außenschicht des Elektrodenkörpers liegt also ein Gradient der Werte einer physikalischen Eigenschaft, wie beispielsweise der Härte, vor, der durch die dazwischen eingesetzte Zwischenschicht "abgemildert" wird. In anderen Worten ändert sich durch die Zwischenschicht der Gradient der Werte der physikalischen Eigenschaften der Innen- und Außenschicht nicht, die Zwischenschicht bewirkt aber, dass der "große" Sprung zwischen den Werten der physikalischen Eigenschaften der Innen- und Außenschicht in mindestens zwei "kleine" Sprünge aufgeteilt wird. Letztere weist einen zwischen den Werten der Innen- und Außenschicht liegenden Zwischenwert der jeweiligen physikalischen Eigenschaft auf. Die entsprechende physikalische Größe vollzieht dann keinen einzelnen Sprung, sondern zwei oder mehrere Stufen oder einen Gradienten beim Übergang zwischen den Schichten. Die Spannungen in den Grenzflächen zwischen den Schichten sinken damit und die zum Stand der Technik geschilderten Probleme werden zumindest signifikant reduziert. Damit können auch weitere Probleme, wie Ablöseerscheinungen, Deformation - beispielsweise in Form eines Faltenwurfes - bei Biegung des Elektrodenkörpers oder eine innere Reibung des Elektrodenkörpers reduziert werden.

Bevorzugte Ausführungsformen der Erfindung ergeben sich aus den abhängigen Ansprüchen. Deren Merkmale, Einzelheiten und Vorteile werden ferner in der nachfolgenden Beschreibung an Hand der beigefügten Zeichnung näher erläutert. Diese

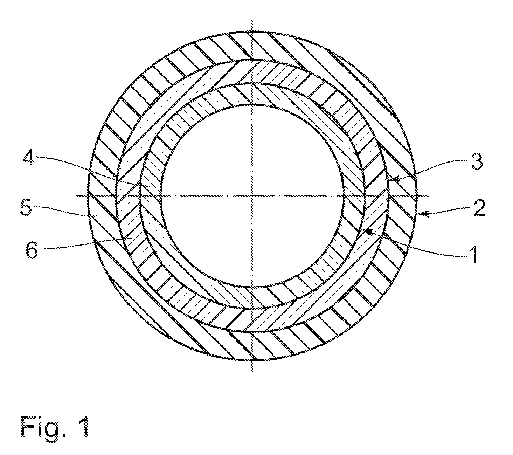

Figur 1 zeigt einen schematischen Schnitt durch einen Elektrodenkörper.

Wie aus Fig. 1 deutlich wird, weist der Elektrodenkörper eine Innenschicht 1 und eine Außenschicht 2 auf. Dazwischen ist eine Zwischenschicht 3 gesetzt. In der gezeigten Ausführungsform bestehen alle Schichten 1, 2 und 3 aus Schläuchen 4, 5, 6. Der die Innenschicht 1 bildende Schlauch 4 kann dabei aus weichem Silikon, der die Außenschicht 2 bildende Schlauch 5 aus hartem Polyurethan und der die Zwischenschicht 3 bildende Schlauch 6 ein mittelharter Polyurethan-Schlauch sein. Die Schläuche 4 bis 6 können eng aufeinanderliegen, wobei dazwischen statt Luft ein Gleitmittel eingebracht werden kann. Auch eine Verklebung der Schläuche 4 bis 6 aneinander ist je nach Anwendungszweck möglich.

Die Innenschicht 1 kann auch beispielsweise aus Copolymeren gebildet sein. Die Außenschicht 2 kann bevorzugt aus PTFE, besonders bevorzugt aus Teflon®, oder Copolymeren, besonders bevorzugt aus Silikon-Urethan Copolymere, gebildet sein. In einer weiteren Variante ist zwischen der Innenschicht 1 und der Außenschicht 2 ein Primer als Zwischenschicht 3 eingesetzt. Dieser Primer ist dann vorteilhaft, wenn anstatt von Schläuchen Beschichtungen eingesetzt werden können.

Schließlich können auch Beschichtungen mit unterschiedlichen Werten der jeweiligen Eigenschaften des Werkstoffes zur Realisierung herangezogen werden. Die folgende Tabelle gibt in diesem Zusammenhang eine beispielhafte Auswahl für verschiedene Ausbildungen der Schichten 1 bis 3:

| **Beispiel** | **Innenschicht 1** | **Zwischenschicht 2** | **Außenschicht 3** |
|---|---|---|---|
| 1 | Silikonschlauch weich | PU-Schlauch weich | PU-Schlauch hart |
| 2 | Silikonschlauch weich | PU-Beschichtung weich | PU-Schlauch hart |
| 3 | Silikonschlauch weich | PU-Schlauch weich (Härte 80 AE) | PU-Beschichtung hart (Härte 55 D, Dicke 20µm) |
| 4 | Silikonschlauch weich | PU-Beschichtung weich (Härte 80 A) | ein oder mehrere PU-Beschichtungen - nach außen härter werdend (Härten 60 A, 55D) |
| 5 | Silikonschlauch weich | Haftbeschichtung aus Copolymer | PU-Beschichtung hart |

Folgende Anmerkungen sind zu den vorgenannten Beispielen zu geben:

Beim Beispiel 3 wirkt die äußere harte PU-Beschichtung vor dem bei einem PU-Schlauch mit einer Härte 80 AE größeren Risiko der Degradation und verbessert die Gleiteigenschaften.

Beim Beispiel 4 kann die Innenschicht 1 auch durch einen an sich harten Silikonschlauch gebildet werden, der mit einer gut haftenden, weichen Silikonbeschichtung versehen ist. Darauf wird eine gut haftende, etwas weniger weiche PU-Beschichtung und eine harte und abriebfeste PU-Außenschicht aufgetragen. 1

## Patentansprüche

1. Elektrodenvorrichtung zum physiologischen Einsatz insbesondere in der Kardiologie mit einem aus elektrisch isolierenden, elastischen Materialien schichtweise aufgebauten Elektrodenkörper, **dadurch gekennzeichnet, dass**
- zwischen einer Innenschicht (1) und einer Außenschicht (2) des Elektrodenkörpers ein Gradient der Werte einer physikalischen Eigenschaft der Schichten (1, 2) vorliegt, und
- zwischen Innenschicht (1) und Außenschicht (2) mindestens eine Zwischenschicht (3) eingesetzt ist, die mindestens einen zwischen den Werten der Außen- und Innenschicht liegenden Zwischenwert dieser physikalischen Eigenschaft aufweist.

2. Elektrodenvorrichtung nach Anspruch 1, **dadurch gekennzeichnet, dass** die physikalische Eigenschaft die Härte, Elastizität, Biege- oder Torsionssteifigkeit ist.

3. Elektrodenvorrichtung nach Anspruch 1 oder 2, **dadurch gekennzeichnet, dass** die Schichten (1, 2, 3) mit unterschiedlichen Werten von physikalischen Eigenschaften durch Schläuche (4, 5, 6), Beschichtungen oder Kombinationen davon gebildet sind.

4. Elektrodenvorrichtung nach einem der vorgenannten Ansprüche, **dadurch gekennzeichnet, dass** die Innenschicht (1) durch einen weichen Silikonschlauch (4) und die Zwischenschicht (3) und Außenschicht (2) durch demgegenüber nach außen abgestuft härtere Polyurethan-Schläuche (5, 6) oder -Beschichtungen gebildet sind.

5. Elektrodenvorrichtung nach einem der vorgenannten Ansprüche, **dadurch gekennzeichnet, dass** bei einer Ausbildung der Schichten (1, 2, 3) in Form von ineinander sitzenden Schläuchen (4, 5, 6) diese miteinander verklebt sind.
